# EUROPEAN PATENT APPLICATION

(11) **EP 2 984 978 A1**
(43) Date of publication of application: **17.02.2016**
(21) Application number: 13881301.9
(22) Date of filing: 05.11.2013
(51) Int. Cl.: A47L 9/10

(54) **HYDRODYNAMICALLY OPERATING VACUUM CLEANER THAT DOES NOT USE A FILTER OR A BAG IN ORDER TO CARRY OUT THE DECONTAMINATION**

(30) Priority: 05.04.2013 MX 2013000162 U
(71) Applicant: Vacman Internacional, S.A. De C.V., 52740 Ocoyoacac (MX)
(72) Inventor: SALTIEL-GONSEN, Alejandro, 52740 Ocoyoacac Edo. de México (MX); DELA FUENTE-PAREDES, Héctor, Alejandro, 52740 Ocoyoacac Edo. de México (MX); MELGAREJO-ARZATE, Armando, 52740 Ocoyoacac Edo. de México (MX)
(74) Representative: Mannion, Daniel James
(86) International application number: PCT/IB2013/002444
(87) International publication number: WO 2014/162165

(57) **Abstract**

The invention relates to a hydrodynamically operating vacuum cleaner that does not use a filter or a bag in order to carry out the decontamination, said vacuum cleaner comprising: a casing; an electric motor; a switch for switching the electric motor on and off; a handle cover; a power outlet for a motorised brush; a plurality of holders for a water container; a cable-holding hook; a turbine and a turbine nut; an air inlet; a water container; and a rolling support, where the water container is made of plastic materials mixed with silver on a scale of between 1% to 6%.

## Description

### FIELD OF THE INVENTION

The present invention relates to vacuum cleaners utilized to clean and decontaminate enclosed premises, and more particularly it relates to a hydrodynamically operating vacuum cleaner that does not use a filter or a bag in order to carry out the decontamination.

### BACKGROUND OF THE INVENTION

In the technical field of the vacuum cleaners, these operate in a cleaning mode with vacuum and a cleaning mode with filter and/or bag. Vacuum cleaners commonly found described in the prior art comprise a filtration unit employed to separate the dust particles found in an airflow. The dust particles not separated from the principal airflow are collected by a subsequent filter. As may be observed, this type of vacuum cleaner employs at least two different filters and on one thereof becoming obstructed by the fine dust it permits the passage of said dust towards the other filter and finally into the interior of the motorized unit of the vacuum cleaner. In some models, to prevent part of this fine dust returning to the environment, said vacuum cleaners have a third filter at the egress of the airflow. Nevertheless, this type of vacuum cleaner involves a high differential pressure during vacuum cleaning, resulting in a high energy consumption and a loud noise during the operation thereof.

One of the principal problems which the vacuum cleaners of the prior art present is that when the bag becomes full, the vacuum cleaner loses the capacity thereof to pick up. The capacity of a vacuum cleaner to store dust is restricted by the size of the bag, it being inconvenient for the user to have to replace the bag every time that the latter becomes full or deteriorates. Similarly, once the bag becomes saturated it allows fine dust to pass towards the motor and the exterior.

As a consequence of said problems, the working life of said vacuum cleaners is very limited by virtue of the fact that the dust residues which pass to the motor damage it, and with the passage of time the damage becomes greater and it becomes non-viable economically to provide a refurbishment service to recover the lost efficiency.

### OBJECTS OF THE INVENTION

An object of the present invention is to provide a hydrodynamically operating vacuum cleaner that does not use a filter or a bag in order to carry out the decontamination, permitting the decontamination of the air which it sucks in by means of the incorporation of silver in those elements of the vacuum cleaner in direct contact with the internal flow of fluids (water/air).

Another object of the present invention is to provide a hydrodynamically operating vacuum cleaner that does not use a filter or a bag in order to carry out the decontamination, permitting thorough cleaning of the parts forming the space or premises which the user requires to clean.

A further object of the present invention is to provide a hydrodynamically operating vacuum cleaner that does not use a filter or a bag in order to carry out the decontamination, permitting sucking in of both dust particles and water.

A yet further object of the present invention is to provide a hydrodynamically operating vacuum cleaner that does not use a filter or a bag in order to carry out the decontamination, having a long working life without requiring maintenance on the part of the user.

### BRIEF SUMMARY OF THE INVENTION

The hydrodynamically operating vacuum cleaner of the present invention does not use bags nor filters in comparison with the vacuum cleaners described in the prior art, by virtue of the fact that the hydrodynamic system permits both the contaminated air circulating in the environment and the impurities contained in the premises to be sucked in in an efficient manner, preventing in this manner diverse respiratory problems such as asthma and allergies caused by mites (dust mites) and environmental dust.

The present hydrodynamically operating vacuum cleaner sucks in the dirty and contaminated air during the process of cleaning enclosed premises. Said air passes through a water container, with water, retaining the solids entering together with the air, and immediately a turbine permits the passage of the air alone, leaving in the water container both the water and the solid materials. The air which subsequently egresses from the vacuum cleaner does not contain germs or bacteria.

The foregoing is due to the fact that the water container is manufactured from polymeric materials which may be mixed with a concentration of silver in a range from 1 % to 6 %, by virtue of the fact that the natural bactericidal properties of the silver permit attacking harmful bacteria and germs arising from the air circulating within the vacuum cleaner, together with the impurities housed in the same, consequently in this manner purified air is obtained following the cleaning of the aforementioned premises.

### BRIEF DESCRIPTION OF THE FIGURES

By means of diverse figures below there may be observed the structural disposition of a particularly preferred embodiment of a hydrodynamically operating vacuum cleaner of the present invention, together with the parts and accessories comprising it:
- Figure 1A: shows a front view of the hydrodynamically operating vacuum cleaner of the present invention.
- Figure 1B: shows a right-hand side view of the hydrodynamically operating vacuum cleaner of the present invention.
- Figure 1C: shows a rear view of the hydrodynamically operating vacuum cleaner of the present invention.
- Figure 2: shows a left-hand cross-sectional side view of the hydrodynamically operating vacuum cleaner of the present invention, wherein the internal elements comprising it may be observed.
- Figure 3: shows some accessories which may be mounted on the hydrodynamically operating vacuum cleaner of the present invention.

### DETAILED DESCRIPTION OF THE INVENTION

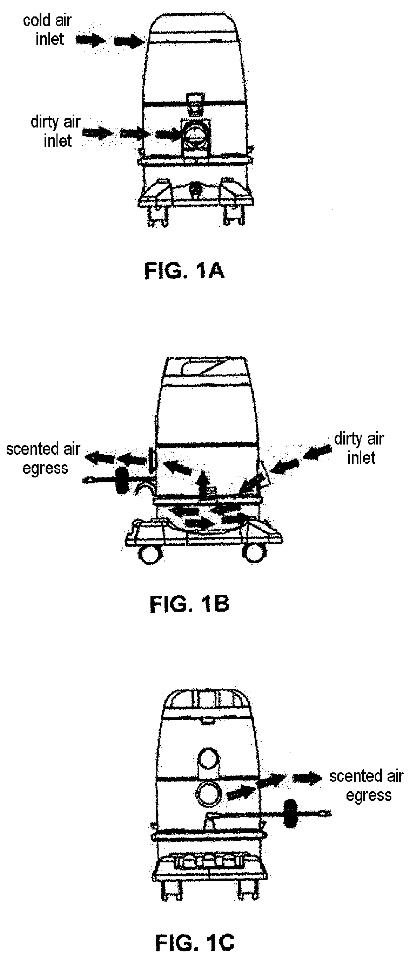

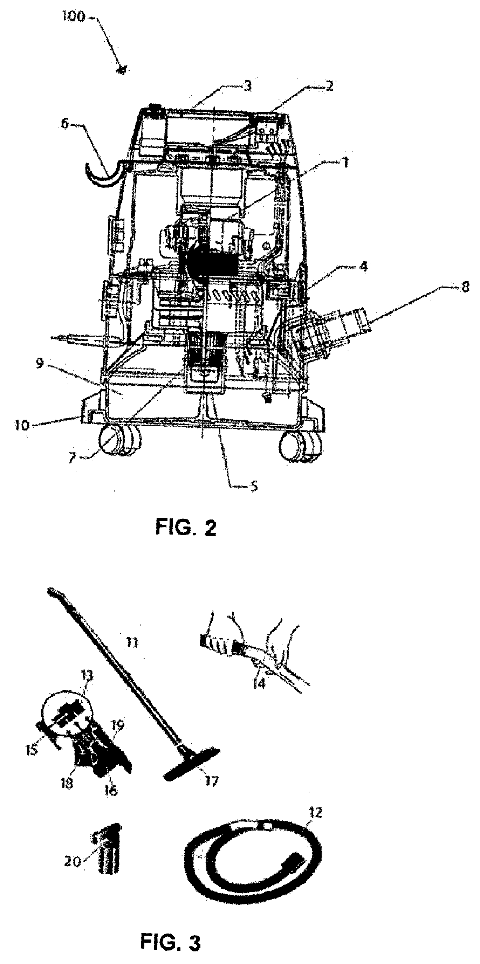

Making reference to the appended figures, and more specifically to Figures 1A, 1B, 1C and 2, therein there is shown a hydrodynamically operating vacuum cleaner 100 that does not use a filter or a bag in order to carry out the decontamination, constructed in accordance with a preferred embodiment of the present invention which shall be considered to be illustrative but not limitative of the same, wherein said hydrodynamically operating vacuum cleaner 100 comprises a bypass type universal electric motor 1 having a frequency of 50/60 Hz at 120 volts and 7.5 amperes consumption at an operational speed of 16 000 rpm, a switch 2; a handle cover 3; a power outlet 4 for a motorized brush; holders 5 for the water container; a cable holding hook 6; a turbine and a turbine nut 7; an air inlet 8; a water container 9; and a rolling support 10.

Figure 3 shows in detail accessories of the hydrodynamically operating vacuum cleaner 100 object of the present invention capable of being mounted, in an optional manner by the user and on the basis of operating compatibility. Specifically, there exist and are shown the following accessories:
- A telescopic tube 11
- A suction hose 12
- An accessory holder 13
- A bent handle tube 14
- A brush to wash the turbine 15
- A nozzle for furniture or upholstered surfaces 16
- A brush for floors and walls 17
- A brush for dust or duster 18
- A nozzle for cracks and crevices 19
- An atomizer 20

Figures 1A, 1B and 1C show different views (front, right hand side and rear, respectively) of the hydrodynamically operating vacuum cleaner 100, wherein there is also shown in schematic form, by means of arrows, the flow of the air through said hydrodynamically operating vacuum cleaner 100. The water container 9 is composed of two parts: the base, manufactured with polycarbonate plastic material or copolyester plastic material, and the cover, manufactured with ABS plastic material. Both parts may be mixed with a concentration of silver in a range from 1 % to 6 % of nanosilver, such that on contact with the water the natural bactericidal properties of the silver are transferred.

In order for the hydrodynamically operating vacuum cleaner 100 to operate properly, the water container 9 must be filled with cold water, up to the upper extremity of a central post, with a recommended water quantity of 2.9 liters, that is to say until the extremity of the central post of the water container 9 is covered.

The hydrodynamically operating vacuum cleaner 100 of the present invention having been placed on the water container 9, the air inlet 8 protrudes through a lateral opening. It is important to mention that the edge forming the motor unit (electric motor) of the vacuum cleaner 100 must rest firmly upon an upper slot of the water container 9. The two holders for the water container 5 must be located and secured on the rim of the water container 9.

The vacuum cleaner 100 is mounted on the rolling support 10 and the telescopic tube 11 is inserted into the brush for floors and walls 17, securing said brush 17 with a button. The accessory holder 13 is located on the upper part of the telescopic tube 11. In said accessory holder 13 there are placed the other remaining accessories: the brush to wash the turbine 15, the brush for dust or duster 18, the nozzle for furniture or upholstered surfaces 16 and the nozzle for cracks and crevices 19.

An electric cable is connected feeding electrical energy to the motor 1 by means of the outlet wall socket and the hydrodynamically operating vacuum cleaner 100 is switched on putting the switch 2 into the 'on' position. The rotary extremity of the suction hose 12 is introduced into the air inlet 3 and the bent handle tube 14 is placed into the fixed extremity of said suction hose 12, said hose 12 being connected to the telescopic tube 11.

Having completed the assembly and switched on the present vacuum cleaner 100 with all the accessories thereof, the process of vacuum cleaning and consequently the cleaning of the surfaces is initiated: the suction generated by said vacuum cleaner 100 carries all the dirt, impurities and dust towards the water container 9, wherein they are deposited and mixed with the water in the interior thereof, the solids remaining in the interior of the container 9 whilst the turbine 7 generates an air swirl entering into contact with the surfaces of the container 9 and, prior to reaching the egress, the air is decontaminated. Additionally, a freshener mixture may be poured into the water contained in the water container 9 prior to the egress of the air to provide fresh, clean and decontaminated air (without germs and bacteria) to the place where it is desired to clean and decontaminate.

### VERIFICATION OF THE EFFICIENCY

The air obtained at the egress from the hydrodynamically operating vacuum cleaner, immediately subsequent to the realization of a cleaning operation, is subjected to laboratory tests to measure the degree of contamination with bacteria or germs and to establish a comparison with the surrounding air which has not passed through the hydrodynamically operating vacuum cleaner and to be able to determine the effectiveness of the bactericidal effect.

By virtue of the foregoing, and with the objective of demonstrating the properties and advantages of utilizing impregnation with silver (nanosilver), samples of water containers constructed according to the principles of the present invention were subjected to analysis to determine the efficiency of the aerobic mesophilic organism bacterial reduction during the treatment of domestic air. The analysis was carried out in conformity with the method of the Mexican Official Standard NOM-092-SSA1 1994.

The samples were obtained utilizing the hydrodynamically operating vacuum cleaner of the present invention, the water container whereof having the following characteristics:
- Material of the base: copolyester plastic material (without nanosilver)
- Material of the cover: ABS plastic material mixed with up to 3 % of nanosilver.

The results obtained were the following:

| | | |
|---|---|---|
| Date of analysis | 12/06/2012 | 09/30/2013 |
| Initial viable count | 9.0 cfu* | 70.0 cfu* |
| Surviving cell count | < 1.0 cfu* | < 1.0 cfu* |
| Bacterial reduction (%) | 100 % | 100 % |
| Method | NOM-092-SSA1-1994 | NOM-092-SSA1-1994 |

| | | |
|---|---|---|
| * cfu = colony forming unit | | |

## Claims

1. A hydrodynamically operating vacuum cleaner that does not use a filter or a bag in order to carry out the decontamination of the type comprising: a casing; an electric motor; a switch for switching the electric motor on and off; a handle cover; a power outlet for a motorized brush; a plurality of holders for a water container; a cable-holding hook; a turbine and a turbine nut; an air inlet; a water container; and a rolling support, **characterized in that** the water container is made of plastic materials mixed with silver.

2. The hydrodynamically operating vacuum cleaner as claimed in claim 1, **characterized in that** the water container is formed of two parts: i) a base, being manufactured with a polycarbonate or copolyester plastic material, and ii) a cover, being manufactured with an ABS plastic material, wherein the plastic materials may be mixed with a concentration of silver in a range from 1 % to 6 % with the objective of when the container is in contact with water it transferring thereto the bactericidal properties of the silver, in this manner attacking the germs collected from the circulating air.

3. The hydrodynamically operating vacuum cleaner as claimed in claim 2, **characterized in that** the base of the water container is manufactured with a copolyester plastic material and the cover of the water container is manufactured with an ABS plastic material mixed with a concentration of up to 3 % of silver by weight.

4. The hydrodynamically operating vacuum cleaner as claimed in claims 2 or 3, **characterized in that** once the turbine of the vacuum cleaner is in motion it generates a hydrodynamic force separating the solids from the air and depositing them in the water container.

5. The hydrodynamically operating vacuum cleaner as claimed in claim 4, **characterized in that** once the air has been filtered it is scented with a mixture of essences poured into the water container, such as in this manner to provide fresh, clean and decontaminated air (without germs or bacteria).
